Europäisches Patentamt

(19) European Patent Office

Office européen des brevets

(11) Publication number: **0 194 156**
**B1**

(12) **EUROPEAN PATENT SPECIFICATION**

(45) Date of publication of patent specification: **21.02.90**

(21) Application number: **86301634.1**

(22) Date of filing: **07.03.86**

(51) Int. Cl.⁵: **G 01 N 33/536,**
**G 01 N 33/541,**
**G 01 N 33/543,**
**G 01 N 33/545,**
**G 01 N 33/546,**
**G 01 N 33/555,**
**G 01 N 33/569**

(54) Method of measuring the amount of immune antibody in serum.

(30) Priority: **08.03.85 JP 45897/85**

(43) Date of publication of application:
**10.09.86 Bulletin 86/37**

(45) Publication of the grant of the patent:
**21.02.90 Bulletin 90/08**

(84) Designated Contracting States:
**DE FR GB**

(56) References cited:
**DE-A-2 934 757**
**DE-A-3 006 899**
**DE-A-3 303 793**

(73) Proprietor: **SANKO JUNYAKU CO., LTD.**
**TMM Bldg. 10-6 Iwamoto-cho 1-chome**
**Chiyoda-ku Tokyo 101 (JP)**

(72) Inventor: **Tomizawa, Takayuki**
**26-18-905, Shinjuku 7-chome Shimjuku-ku**
**Tokyo (JP)**

(74) Representative: **Allam, Peter Clerk et al**
**LLOYD WISE, TREGEAR & CO. Norman House**
**105-109 Strand**
**London WC2R 0AE (GB)**

## Description

This invention relates generally to a method of measuring the amount of an immune antibody in blood serum and, more specifically, to a method of measuring the amount of a desired immune antibody in serum which contains one or more immune antibodies inclusive of the desired one.

When a person is infected, immunity will be developed to counter the disease during its progress. The symptoms will then be reduced, and healing will begin. Specific antibodies (IgM antibody, IgA antibody, IgG antibody, etc.) will have been produced and if the patient's serum is examined at this point, the stage of immunity can be identified. Infectious diseases have various conditions of infection; some will progress rapidly, and others will take longer to progress or become chronic. In both cases, IgM antibodies against the disease will be produced in the primary stage of infection. They will gradually be taken over by IgG antibodies. Meanwhile, treatment may be given or the infection may heal naturally. When a person is infected, the IgM antibodies will increase and, when production reaches its peak, the IgM antibodies will gradually decrease. When the infection is healed, the IgM antibodies will disappear.

While the IgM antibodies are decreasing, the IgG antibodies will appear and will remain for a longer period of time. Even while the IgM antibodies are disappearing, the IgG antibodies are being actively produced. However, as diseases vary in their conditions of infection, the IgG antibodies will also be reduced accordingly. Including all other antibodies present under the measurement limit, it is believed that when the specific antibodies are present, many infectious diseases cannot be contracted again; but their presence does not mean the person will not be re-infected. For this reason, it is considered that immunity is no longer present when the antibodies have disappeared, and infection may recur. Therefore, by measuring the antibodies it is possible to detect the present of immunity to the disease. Further, from the detection of IgM antibodies which are produced in the primary stage, it is also possible to determine whether the patient is in the acute stage of infection during which the disease is infectious. Moreover, determination of the amount of IgM antibodies signifies the degree of immunity after recovery from the primary stage of the disease. Consequently, it is significant to measure the IgG antibodies and IgM antibodies separately and quantitatively for various reasons.

However, the conventional serological diagnostic approach is intended to measure all the antibodies together and fails to selectively measure specific antibodies. Laboratory methods have recently been developed which employ a reagent for measuring the IgM and IgG antibodies in radio-immuno assays (RIA) using radioactive isotopes or enzyme-immuno assays (EIA) that use enzyme-linked antibodies for measurement. However, as these methods require complex operations to measure IgM and IgG antibodies, they have not been put to practical use.

DE—A—3006899 (and GB—A—2045431) discloses a method useful, inter alia, for measuring the amount of an immune antibody contained in serum and which can involve the steps of:

(a) providing a first suspension containing a first particulate carrier which has a first particle size and which carries a first substance capable of reacting with said immune antibody;

(b) mixing said serum with said first suspension to react said first substance with said immune antibody and to bind said immune antibody to said first carrier;

(c) providing a second suspension containing a second particulate carrier which has a second particle size smaller than said first particle size and which carries a second substance capable of reacting with said immune antibody, wherein at least one of said first and second substances is capable of selectively reacting only with said immune antibody;

(d) mixing said first carrier obtained in step (b) and having bound immune antibody with said second suspension to react said second substance with said immune antibody and to bind said second carrier to said first carrier in an amount corresponding to the amount of said immune antibody bound to said first carrier;

(e) separating said first carrier together with the second carrier bound thereto from the admixture obtained in step (d) to obtain a third suspension containing the second carrier which has not been bound to said first carrier; and

(f) measuring the amount of said second carrier in said third suspension.

It is suggested that the amount of the second carrier in the third suspension, i.e. step (f), should be accomplished by counting using a counter programmed to recognize particles of a certain size, or by labelling the second carrier e.g. with a radioactive atom, a fluorophore or an enzyme, and then using an appropriate assay.

The present invention provides a method for measuring the amount of an immune antibody e.g. IgM, IgG or other antibody, contained in serum, which method is of the kind set forth above and which is characterized in the step (f), i.e. the measurement of the amount of the second carrier in the third suspension, is accomplished by measuring the tubidity of the third suspension.

The present invention will now be described in detail below.

In the method according to the present invention, two separate first and second suspensions are used for measuring the amount of a target immune antibody contained in serum. The first suspension contains a first particulate carrier having a first particle size, while the second suspension contains a second particulate carrier having a second particle size which is smaller than the first particle size. The first carrier carries a first substance capable of reacting with an immune antibody in serum the amount of which is to

2

be measured. Also, the second carrier carries a second substance capable of reacting with the immune antibody. The first and second substances, in general, are different from each other. It is important that at least one of the first and second substances should selectively react only with the desired immune antibody whose amount is to be measured. Thus, for example, when the first substance used is of a first type which is able to react not only with the target immune antibody (e.g. IgM antibody) but also with other immune antibody or antibodies (e.g. IgA and IgG), the second substance used must be of a second type which reacts only with the target immune antibody (IgM). On the other hand, if the first substance used is of the first type or the second type.

The first type substance may be, for example, an infectious antigen such as a pathogenic organ or its component. The second type substance may be, for example, an anti-antibody. Thus, when the immune antibody to be measured is an IgM antibody, then the anti-antibody is an anti-IgM antibody. If the measurement of an IgG antibody is intended, an anti-IgG antibody is used as the anti-antibody.

As described previously, the first and second substances are carried by particulate carriers. The carrying is preferably by adsorption. The first carrier may be water-insoluble solid particles. Illustrative of suitable first carriers are vital substances such as animal red blood cells, human red blood cells, bacteria, gelatin and polysaccharides; inorganic substances such as kaolin and carbon; and synthetic polymeric substances such as polystyrene. The particle size of the first carrier is preferably 1—10 μm, more preferably 5—8 μm.

The second carrier may be water-insoluble solid particles such as polystyrene or other synthetic polymeric substances, or kaolin, carbon or other inorganic substances. The particle size of the second carrier is preferably 0.05—1 μm, more preferably 0.1—3 μm. The material and kind of the first and second carriers may be suitably selected so that the first and second carriers when in admixture can be separated from each other by a simple method such as by centrifuging, sedimentation, filtration or magnetic separation.

One embodiment according to the present invention thus uses a first particulate carrier supporting thereon, ie sensitized with, an antigen and a second particulate carrier supporting thereon, ie sensitized with, an anti-antibody. The sensitization of the first carrier with the antigen and the sensitization of the second carrier with the anti-antibody may be effected in any known manner. The serum containing the immune antibody to be measured is generally diluted with a 0.005 Mol phosphate buffered saline solution containing 0.1% of bovine serum albumin. The serum sample of interest is first mixed with the first suspension containing the antigen-carrying first carrier to react the immune antibodies contained in the serum with the antigen and to bind the immune antibodies to the first carrier. The antigen-antibody reaction may be performed at room temperature but preferably at 30—37°C.

After the completion of the antigen-antibody reaction, the reaction mixture is preferably separated by centrifuging, for example, into solids and a liquid phase. The centrifuge may be operated, for example at 2000 r.p.m. for 5 min. The solids are then mixed with a sufficient amount of physiological saline solution with agitation for washing. The mixture is again separated centrifugally at 2000 r.p.m. for 5 min and the supernatant liquid is removed. The resultant solids are suspended in an aqueous medium containing 3% sucrose to obtain a specimen containing the first carrier having bound antibodies from the serum.

The thus obtained specimen is mixed with the second suspension containing, for example, 0.01% of the second carrier sensitized with the anti-antibody and suspended in an aqueous medium containing 3% sucrose, thereby to react the antibody carried on the first carrier with the anti-antibody carried on the second carrier and to bind the second carrier to the first carrier in an amount corresponding to the amount of the antibody carried on the first carrier. The reaction may be performed, for example, at 32°C for 10 min.

From the thus obtained mixture, the first carrier with bound second carrier and the first carrier without bound carrier are both removed to obtain a third suspension containing the remainder of the second carrier which has not been bound to the first carrier. This can be obtained, for example, by centrifuging at 1500 r.p.m. for 10 min. Other techniques such as sedimentation, filtration, magnetic separation may also be adopted as desired. The concentration of the second carrier contained in the third suspension is then determined from measurements of its turbidity. Preferably the difference in tubidity between the third suspension and a control third suspension which does not contain a first substance capable of reacting with the immune antibody is determined in order to measure more precisely the amount of the immune antibody contained in the serum.

For example, by using an anti-IgM sensitized suspension as the second suspension, the amount of the IgM antibody in the serum can be measured. When an anti-IgG sensitized suspension is used as the second suspension, the amount of the IgG antibody contained in the serum may be selectively measured.

More specifically, when an anti-IgM sensitized suspension is mixed with the afore-mentioned specimen containing immune antibody-carrying first carrier, the IgM antibody on the first carrier reacts with the anti-IgM on the second carrier so that the anti-IgM-carrying second carrier is bound to and agglutinated with the IgM antibody-carrying first carrier. The centrifugal treatment of the resultant mixture can precipitate the coagulated mass together with the first carrier, thereby to give a supernatant containing the residual anti-IgM-carrying second carrier which remains unreacted. The amount of the anti-IgM-carrying second carrier which has reacted with the IgM antibody can be determined from the density of the second carrier in the supernatant liquid. When another anti-antibody sensitized suspension such as an anti-IgG sensitized suspension is used, then the amount of the anti-IgG-carrying second carrier which has reacted with the IgG antibody can be determined in the same manner as above. Since the amount of the anti-antibody-carrying second carrier which has reacted with the immune antibody carried on the first carrier corresponds to the amount of the immune antibody, the amount of the immune antibody contained in the blood serum can be determined. The amount of the second carrier in the suspension or supernatant may be suitably determined by measuring its turbidity by means of a UV or visible light spectrophotometer.

In a second embodiment according to the present invention, the first carrier supports an anti-antibody, e.g. anti-IgM, while the second carrier supports an antigen for the purpose of measuring the amount of the immune antibody, e.g. IgM antibody, contained in serum. The anti-IgM diluted with 100 times the amount of a 0.15 M phosphatic buffer solution is mixed with a first carrier to adsorb the anti-IgM thereon in a manner known per se, thereby obtaining a first suspension. 0.15 ml of the first suspension is mixed with 0.25 ml of a serum which had been diluted 10—100 times. The mixture is reacted for 10 min at 32°C. The resultant mixture is centrifugally separated at 2000 r.p.m. for 10 min into a precipitate and a supernatant. The precipitate is mixed with a sufficient amount of a physiological saline solution with agitation for washing and the mixture is subjected again to centrifuge at 2000 r.p.m. for 5 min. The thus obtained precipitate, i.e. first carrier containing the IgM antibody which has been contained in the serum, is suspended in 1.5 ml of an aqueous medium containing 3% of sucrose to obtain a specimen. Meanwhile, a second suspension containing 0.01% of a second carrier carrying an antigen such as a pathogenic organ capable of reacting with the IgM antibody is prepared using a 3% sucrose aqueous solution as suspension medium. The second suspension is then mixed with the above specimen and the mixture is allowed to react at 32°C for 10 min. The resultant mixture is separated for example by centrifuge at 1500 r.p.m. for 10 min, into a precipitate and a supernatant. The tubidity of the supernatant is measured for the determination of the amount of the IgM contained in the serum. By using an anti-IgG in place of the anti-IgM, the above procedure can selectively measure the amount of the IgG antibody in the serum.

As will be understood from the foregoing, the present invention makes it possible to selectively measure a desired one of the immune antibodies produced to counter various antigens quantitatively in a simple manner. For example, IgM and IgG antibodies can be measured separately. The method of the present invention is applicable to measure a variety of antibodies against various antigens, including pathogenic microorganisms, protozoa and viruses, causing such deseases as filariasis, moniliasis, leptospirosis, mycoplasmosis, aspergillosis, coccidioidomycosis, tuberculosis, syphilis, rubella, toxoplasmosis, herpes, cytomegalosis, adult T-cell leukemia and acquired immune defficiency syndrome.

The following examples will further illustrate the present invention.

Example 1
Measurement of Syphilis IgM and IgG Antibodies

(1) Preparation of First Suspension:

Treponema pallidum (hereinafter referred to as T.P.), an antigen, is inoculated into a rabbit's testicle, and a biological culture is obtained. T.P. is then extracted from the testicle after about ten days from the start of the culture and is fractionated by centrifugation. It is treated with a sound wave of 10 kilocycles for 10 min to obtain a T.P. fluid. A fixed blood cell fluid from a sheep is prepared and mixed with the T.P. fluid to obtain a first suspension containing the sheep blood cells sensitized with T.P.

(2) Preparation of Second Suspension:

A latex containing particulate polystyrene having a particle size of 0.2 μm is diluted with 0.005 M phosphatic buffer saline solution to obtain a suspension containing 0.25% of particulate polystyrene. The suspension is divided into halves to which are mixed anti-human-IgM and anti-human-IgG, respectively, thereby obtaining two kinds of second suspensions containing 0.01% of anti-IgM sensitized particulate polystyrene and 0.01% of anti-IgG sensitized particulate polystyrene, respectively.

(3) Preparation of Serum Sample:

Rabbit's IgG serum is diluted with 0.005 M phosphatic buffer saline solution containing 0.1% of bovine albumin to obtain a diluent containing 1% of the rabbit's IgG serum. A human serum to be tested is diluted with 9 times the volume of the diluent to obtain a serum sample A. The serum sample A is further diluted with 9 times the volume of the diluent to obtain a serum sample B.

(4) Measurement:

Serum sample A (0.25 ml) is poured into test tubes I and II and serum sample B (0.25 ml) is poured into test tubes III and IV. To each of the test tubes I and III is added 0.15 ml of the first suspension, while to each of the test tubes II and IV is added 0.15 ml of a control suspension containing the same blood cells as above with the exception that the cells are not sensitized with T.P. antigen. Each test tube is maintained at 32°C for 10 min and the resulting mixture in the test tube is separated into a precipitate and a supernatant by centrifuge. The precipitate is mixed with a physiological saline solution and the mixture is agitated for washing the precipitate, followed by centrifugation at 2000 r.p.m. for 5 min. After the removal of the supernatant, 1.5 ml of an aqueous medium containing 3% of sucrose is added to the remaining sediment in each test tube to obtain a suspended specimen. Into the test tubes I and II is then added 1.5 ml of the anti-IgM sensitized second suspension while 1.5 ml of the anti-IgG sensitized second suspension is added to each of the test tubes III and IV. The test tubes I—IV are then maintained at 32°C for 10 min, followed by centrifugation at 1500 r.p.m. for 10 min. The turbidity of each supernatant is measured by means of a spectrophotometer (wavelength: 660 nm).

## Example 2
### Measurement of Rubella IgM and IgG Antibodies

(1) Preparation of First Suspension:

Anti-human IgG and anti-human IgM are each diluted with 9 times the volume of 0.005 M phosphatic buffer saline solution, to which is added an equi-volume of a 2.5% sheep fixed blood cell fluid in 0.005 M phosphatic buffer saline solution, thereby to obtain two kinds of first suspensions containing the sheep blood cells sensitized with anti-IgG and anti-IgM, respectively.

(2) Preparation of Second Suspension:

A latex containing particulate polystyrene having a particle size of 0.2 μm is diluted with 0.005 M phosphatic buffer saline solution to obtain a suspension containing 2.5% of the particulate polystyrene, with which is mixed an equi-volume of a 0.005 M phosphatic buffer saline solution containing 2.5% of refined rubella virus (antigen). The mixture is then maintained at 32°C for 10 min, followed by centrifugation at 10000 r.p.m. for 20 min. The precipitate thus obtained is washed and suspended in a 0.005 M phosphatic buffer saline solution containing 0.1% of bovine serum albumin, thereby to obtain a second suspension sensitized with the antigen.

(3) Preparation of Serum Sample:

Rabbit's IgG serum is diluted with 0.005 M phosphatic buffer saline solution containing 0.1% of bovine albumin to obtain a diluent containing 1% of the rabbit's IgG serum. A human serum to be tested is diluted with 9 times the volume of the diluent to obtain a serum sample A. The serum sample A is further diluted with 9 times the volume of the diluent to obtain a serum sample B.

(4) Measurement:

Serum sample A (0.25 ml) is poured into test tubes I and II and the serum sample B (0.25 ml) is poured into test tubes III and IV. To each of the test tubes I and III is added 0.15 ml of IgM-sensitized first suspension and IgG-sensitized first suspension, respectively, while to each of the test tubes II and IV is added 0.15 ml of a control suspension containing the same blood cells as above with the exception that the cells are not sensitized with the anti-IgM or anti-IgG. Each test tube is maintained at 32°C for 10 min and the resulting mixture in the test tube is separated into a precipitate and a supernatant by centrifuge. The precipitate is mixed with a physiological saline solution and the mixture is agitated for washing the precipitate, followed by centrifugation at 2000 r.p.m. for 5 min. After the removal of the supernatant, 1.5 ml of an aqueous medium containing 3% of sucrose is added to the remaining sediment in each test tube to obtain a suspended specimen. Into the test tubes I—IV is then added 1.5 ml of the antigen-sensitized second suspension. The test tubes I—IV are then maintained at 32°C for 10 min, followed by centrifugation at 1500 r.p.m. for 10 min. The turbidity of each supernatant is measured by means of a spectrophotometer at a wavelength of 660 nm.

## Examples 3—5

In the same manner as described in Example 1, IgM and IgG antibodies of toxoplasmosis (Example 3), cytomegalosis (Example 4) and herpes (Example 5) in human serum samples are measured.

The results obtained in Examples 1—5 are summarized in Table 1.

TABLE 1

| Desease | State of Subject | Evaluation by Usual Method | Evaluation by Inventive Method | |
| --- | --- | --- | --- | --- |
| | | | IgM Antibody Titer | IgG Antibody Titer |
| Syphilis | Chancre stage | − | 15 | 0 |
| | Primary | + | 55 | 15 |
| | Early | ++ | 30 | 350 |
| | Latent | ++ | 0 | 125 |
| | Cured | − | 0 | 0 |
| | Normal | − | 0 | 0 |
| Rubella | Rash | − | 15 | 5 |
| | Convalascent | + | 5 | 250 |
| | Past | + | 0 | 150 |
| | Uninfection | − | 0 | 0 |
| Toxo-plasmosis | Symptom | + | 20 | 130 |
| | Past | + | 0 | 200 |
| | Uninfection | − | 0 | 0 |
| Herpes | Symptom | + | 30 | 280 |
| | Past | + | 0 | 230 |
| | Uninfection | − | 0 | 0 |
| Cytome-galosis | Symptom | + | 10 | 180 |
| | Past | + | 0 | 400 |
| | Uninfection | − | 0 | 0 |

EP 0 194 156 B1

### Example 6

Example 1 is repeated in the same manner as described except that gelatin particles having a particle size of about 5 μm are used as the carrier for T.P. in place of the sheep blood cells. The gelatin particles are prepared in accordance with the method disclosed in Japanese Published Unexamined Patent Application No. 57—153,658.

### Example 7

Example 2 is repeated in the same manner as described except that commercially available IgM-sensitized immuno bead fluid and IgG-sensitized immuno bead fluid (both manufactured by Bio-Rad Laboratories Inc.) are used as the IgM-sensitized and IgG-sensitized second suspensions, respectively.

### Examples 8—10

In the same manner as described in Example 6, IgM and IgG antibodies of toxoplasmosis (Example 8), cytomegalosis (Example 9) and herpes (Example 10) in human serum samples are measured.

The results obtained in Examples 6—10 are summarized in Table 2. In Tables 1 and 2, the terms "IgM antibody titer" and "IgG antibody titer" are intended to refer to the difference in tubidity (optical density measured by the spectrophotometer) between the sample and control. For example, the IgM and IgG antibody titers in Example 1 represent $(OD_{II}—OD_I) \times 1000$ and $(OD_{IV}—OD_{III}) \times 1000$, respectively, where $OD_I$ to $OD_{IV}$ are optical densities of the supernatants in test tubes I to IV, respectively.

7

TABLE 2

| Disease | State of Subject | Evaluation by Usual Method | Evaluation by Inventive Method | |
|---|---|---|---|---|
| | | | IgM Antibody Titer | IgG Antibody Titer |
| Syphilis | Chancre stage | − | 20 | 0 |
| | Primary | + | 60 | 10 |
| | Early | ++ | 25 | 350 |
| | Latent | ++ | 0 | 100 |
| | Cured | − | 0 | 0 |
| | Normal | − | 0 | 0 |
| Rubella | Rash Stage | − | 25 | 5 |
| | Convalascent | + | 5 | 200 |
| | Past | + | 0 | 150 |
| | Uninfection | − | 0 | 0 |
| Toxo-plasmosis | Symptom Stage | + | 20 | 150 |
| | Past | + | 0 | 150 |
| | Uninfection | − | 0 | 0 |
| Herpes | Symptom Stage | + | 20 | 250 |
| | Past | + | 0 | 250 |
| | Uninfection | − | 0 | 0 |
| Cytome-galosis | Symptom Stage | + | 5 | 170 |
| | Past | + | 0 | 370 |
| | Uninfection | − | 0 | 0 |

# EP 0 194 156 B1

**Claims**

1. A method of measuring the amount of an immune antibody contained in serum, comprising the steps of:

(a) providing a first suspension containing a first particulate carrier which has a first particle size and which carries a first substance capable of reacting with said immune antibody;

(b) mixing said serum with said first suspension to react said first substance with said immune antibody and to bind said immune antibody to said first carrier;

(c) providing a second suspension containing a second particulate carrier which has a second particle size smaller than said first particle size and which carries a second substance capable of reacting with said immune antibody, wherein at least one of said first and second substances is capable of selectively reacting only with said immune antibody;

(d) mixing said first carrier obtained in step (b) and having bound immune antibody with said second suspension to react said second substance with said immune antibody and to bind said second carrier to said first carrier in an amount corresponding to the amount of said immune antibody bound to said first carrier;

(e) separating said first carrier together with the second carrier bound thereto from the admixture obtained in step (d) to obtain a third suspension containing the second carrier which has not been bound to said first carrier; and

(f) measuring the amount of said second carrier in said third suspension; characterized in that step (f) is accomplished by measuring the turbidity of said third suspension.

2. A method according to Claim 1, wherein one of said first and second substances is an anti-antibody and the other one is an antigen.

3. A method according to any preceding claim, wherein said immune antibody is an IgM, IgA or IgG antibody.

4. A method according to Claim 3 when appendant to Claim 2, wherein said anti-antibody is an anti-human-IgA, anti-human-IgM or anti-human-IgG.

5. A method according to any preceding claim, wherein said first and second particle sizes are 1—10 µm and 0.05—1 µm, respectively.

6. A method according to Claim 5, wherein said first and second particle sizes are 5—8 µm and 0.1—0.3 µm, respectively.

7. A method according to any preceding claim, wherein step (e) is effected by centrifuging.

8. A method according to any preceding claim, wherein said first carrier is a water-insoluble material selected from red blood cells, bacteria, gelatin, polysaccharides, synthetic polymeric substances, kaolin and carbon.

9. A method according to any preceding claim, wherein said second carrier is a water-insoluble material selected from carbon, kaolin and synthetic polymeric substances.

10. A method according to any preceding claim, wherein step (d) includes: separating the reaction mixture obtained in step (b) into a precipitate and a liquid phase; recovering said precipitate and washing same with an aqueous wash solution; suspending said washing precipitate with an aqueous medium to obtain a suspended liquid; and mixing said suspended liquid with said second suspension.

11. A method according to any preceding claim, wherein said immune antibody is one induced by filariasis, moniliasis, leptospirosis, mycoplasmosis, aspergillosis, coccidioidomycosis, tuberculosis, syphilis, rubella, toxoplasmosis, herpes, cytomegalosis, adult T-cell leukemia or acquired immune deficiency syndrome.

**Patentansprüche**

1. Verfahren zum Messen der Menge eines Immunantikörpers im Serum, bestehend aus den Verfahrensschritten:

a) Bilden einer ersten Suspension, die einen ersten korpuskularen Träger enthält, der eine erste Teilchengröße hat und eine erste Substanz trägt, die in der Lage ist, mit dem Immunantikörper zu reagieren;

b) Mischen des Serums mit der ersten Suspension, um die erste Substanz mit dem Immunantikörper zur Reaktion zu bringen und den Immunantikörper an den ersten Träger zu binden;

c) Bilden einer zweiten Suspension, die einen zweiten korpuskularen Träger enthält, der eine zweite Teilchengröße hat, die größer ist als die erste Teilchengröße und eine zweite Substanz trägt, die in der Lage ist, mit dem Immunantikörper zu reagieren, wobei zumindest eine der beiden Substanzen in der Lage ist, selektiv nur mit dem Immunantikörper zu reagieren;

d) Mischen des ersten, nach Verfahrensschritt (b) gewonnenen Trägers, der Immunantikörper mit der zeiten Suspension gebunden hat, so daß die zweite Substanz mit dem Immunantikörper reagiert und der zweite Träger an den ersten Träger in der Menge gebunden wird, die der Menge des Immunantikörpers entspricht, die an den ersten Träger gebunden ist;

e) Abscheiden des ersten Trägers zusammen mit dem zweiten daran gebundenen Träger aus der im Verfahrensschritt (d) gewonnenen Zumischung, um eine dritte Suspension zu erhalten, die den zweiten

9

Träger enthält, der nicht an den ersten Träger gebunden wurde, und

f) Mesen der Menge des zweiten Trägers in der dritten Suspension, dadurch gekennzeichnet, daß der Verfahrensschritt (f) durchgeführt wird, indem die Trübheit der dritten Suspension gemessen wird.

2. Verfahren nach Anspruch 1, dadurch gekennzeichnet, daß eine der ersten und zweiten Substanzen ein Antikörper und die andere ein Antigen ist.

3. Verfahren nach einem der vorhergehenden Ansprüche 1 und 2, dadurch gekennzeichnet, daß der Immunantikörper ein IgM-, IgA- oder IgG-antikörper ist.

4. Verfahren nach Anspruch 3 bei dessen Rückbeziehung auf Anspruch 2, dadurch gekennzeichnet, daß der Antikörper ein Antihuman-IgA, Antihuman-IgM oder Antihuman-IgG ist.

5. Verfahren nach einem der vorhergehenden Ansprüche 1 bis 4, dadurch gekennzeichnet, daß die erste und die zweite Teilchengröße zwischen 1—10 µm bzw. 0,05—1 µm liegen.

6. Verfahren nach Anspruch 5, dadurch gekennzeichnet, daß die erste und die zweite Teilchengröße zwischen 5—8 µm bzw. 0,1—0,3 µm liegen.

7. Verfahren nach einem der vorhergehenden Ansprüche 1 bis 6, dadurch gekennzeichnet, daß der Verfahrensschiff (e) durch Zentrifugieren durchgeführt wird.

8. Verfahren nach einem der vorhergehenden Ansprüche 1 bis 7, dadurch gekennzeichnet, daß als erster Träger ein aus roten Blutzellen, Bakterien, Gallerte, Polysacharide, synthetische polymere Substanzen, Kaolin und Kohlenstoff gewähltes wasserunlösliches Material verwendet wird.

9. Verfahren nach einem der vorhergehenden Ansprüche 1 bis 8, dadurch gekennzeichnet, daß als zweiter Träger ein aus Kohlenstoff, Kaolin und synthetischen polymeren Substanzen gewähltes wasserunlösliches Material verwendet wird.

10. Verfahren nach einem der vorhergehenden Ansprüche 1 bis 9, dadurch gekennzeichnet, daß der Verfahrensschritt (d) Folgendes umfaßt: Trennen des im Verfahrensschritt (b) gewonnen Reaktionsgemisches in eine Ausfällung und eine flüssige Phase; Rückführen der Ausfällung und Waschen derselben mit einer wässrigen Waschlösung; Aufschwemmen der gewaschenen Ausfällung mit einem wässrigen Lösungsmittel zur Gewinnung einer aufgeschwemmten Flüssigkeit und Mischen der aufgeschwemmten Flüssigkeit mit der zweiten Aufschwemmung.

11. Verfahren nach einem der vorhergehenden Ansprüche 1 bis 10, dadurch gekennzeichnet, daß es sich bei dem Immunantikörper um einen solchen handelt, der durch Filariasis, Candidose (Monoliasis), Leptospirosis, Mykoplasmosis, Aspergillose, Coccidioidomycosis, Tuberkulose, Syphilis, Röteln, Toxoplasmose, Herpes, Zytomegalie, Erwachsenen-T-Zellen Leukämie oder Aids induziert wurde.

**Revendications**

1. Procédé pour mesurer la quantité d'un anticorps immun contenu dans un sérum, comprenant les étapes qui consistent:

(a) à préparer une première suspension contenant un premier support particulaire qui a une première granulométrie et qui porte une première substance capable de réagir avec ledit anticorps immun;

(b) à mélanger ledit serum avec ladite premiére suspension pour faire réagir ladite première substance avec ledit anticorps immun et pour lier ledit anticorps immun audit premier support;

(c) à préparer une seconde suspension contenant un second support particulaire qui a une seconde granulométrie plus faible que ladite première granulométrie et qui porte une seconde substance capable de réagir avec ledit anticorps immun, l'une au moins desdites première et seconde substances étant capable de réagir sélectivement seulement avec ledit anticorps immun;

(d) à mélanger ledit premier support obtenu dans l'étape (b) et comportant un anticorps immun lié avec ladite seconde suspension pour faire réagir ladite seconde substance avec ledit anticorps immun et pour lier ledit second support audit premier support en une quantité correspondant à la quantité dudit anticorps immun lié audit premier support;

(e) à séparer ledit premier support ainsi que le second support qui est lié à celui-ci d'avec le mélange obtenu en l'étape (d) pour obtenir une troisième suspension contenant le second support qui n'a pas été lié audit premier support; et

(f) à mesurer la quantité dudit second support dans ladite troisième suspension; caractérisé en ce que l'étape (f) est effectuée par la mesure de la turbidité de ladite troisième suspension.

2. Procédé selon la revendication 1, dans lequel l'une première et desdites seconde substances est un anti-anticorps et l'autre est un antigène.

3. Procédé selon l'une quelconque des revendications précédentes, dans lequel ledit anticorps immun est un anticorps IgM, IgA ou IgG.

4. Procédé selon la revendication 3, lorsqu'elle se rapporte à la revendication 2, dans lequel ledit anti-anticorps est une anti-IgA humaine, une anti-IgM humaine ou une anti-IgA humaine, une anti-IgM humaine ou une anti-IgG humaine.

5. Procédé selon l'une quelconque des revendication précédentes, dans lequel ledites première et seconde granulometries sont respectivement de 1—10 m et de 0,05—1 m.

6. Procédé selon la revendication 5, dans lequel lesdites premiere et seconde granulométries sont respectivement de 5—8 m et de 0,1—0,3 m.

7. Procédé selon l'une quelconque des revendication précédentes, dans lequel l'étape (e) est effectuée par centrifugation.

8. Procédé selon l'une quelconque des revendication précédentes, dans lequel ledit premier support est un materiau insoluble dans l'eau choisi parmi les globules rouges, les bactéries, la gélatine, les polysaccharides, les substances polymères synthétiques, le kaolin et le carbone.

9. Procédé selon l'une quelconque des revendications précédentes, dans lequel ledit second support est un matériau insoluble dans l'eau choisi parmi le carbone, le kaolin et les substances polymères synthétiques.

10. Procédé selon l'une quelconque des revendications précédentes, dans lequel l'étape (d) inclut: la séparation du mélange réactionnel obtenu dans l'étape (b) en un précipité et une phase liquide; la récupération dudit précipité et son lavage avec une solution de lavage aqueuse; la mise en suspension dudit précipité lavé avec un milieu aqueux pour obtenir un liquide en suspension; et le mélange dudit liquide en suspension avec ladite suspension.

11. Procédé selon l'une quelconque des revendication précédentes, dans lequel ledit anticorps immun est un anticorps produit par la filariose, la candidose, la leptospirose, la mycoplasmose, l'aspergillose, le granulome coccidioïdien, la tuberculose, la syphilis, la rubeole, la toxoplasmose, l'herpès, la cytomégalose, la leucémie des lymphocytes T de l'adulte ou le syndrome immunodéficitaire acquis.